# EUROPEAN PATENT SPECIFICATION

(11) **EP 3 673 940 B1**
(45) Date of publication and mention of the grant of the patent: **16.08.2023**
(21) Application number: 19216542.1
(22) Date of filing: 16.12.2019
(51) Int. Cl.: A61M 13/00, A61M 16/00, A61M 5/44, A61M 5/00, A61M 5/165, A61M 5/168

(54) **A DEVICE FOR THE ADMINISTRATION OF CARBOXYTHERAPY**
VORRICHTUNG ZUR VERABREICHUNG EINER CARBOXYTHERAPIE
DISPOSITIF D'ADMINISTRATION DE CARBOXYTHÉRAPIE

(30) Priority: 28.12.2018 IT 201800021349
(43) Date of publication of application: 01.07.2020
(73) Proprietor: Acea Medica S.r.l., 20144 Milano (IT)
(72) Inventor: TASSI, Alessandro, 20143 Milano (IT)
(74) Representative: Lunati & Mazzoni S.r.L.

(56) References cited:
- EP-A1- 1 979 029
- WO-A1-2007/034522
- KR-B1- 101 739 898

## Description

The present invention relates to a device for the administration of carboxytherapy of the type specified in the preamble of the first claim. Similar devices are described in patent documents KR-B-101739898 and EP-A-1979029.

Carboxytherapy is a known therapy. It is a non-surgical aesthetic treatment that involves the subcutaneous administration of carbon dioxide (CO₂) in its gaseous state. Carboxytherapy is mainly used for the treatment of the following imperfections or diseases: treatment of cellulite and localized adiposities, treatment of skin problems before and after surgery, revitalization of skin areas, vulvar rejuvenation, improvement of tissue oxygenation, improvement of circulation, treatment of arterial vessel dilation, treatment of arterial disease, treatment of microcirculatory stasis due to medium venous insufficiency in angiology, treatment of rheumatic diseases and periarthritis in orthopaedics.

To carry out these administrations, the carbon dioxide is stored compressed in a special container, and sent, by means of special valves and ducts, to a needle that, for treatment, is inserted in the patient subcutaneously.

The prior art described comprises some significant drawbacks.

In particular, the subcutaneous administration of carbon dioxide is considered, by some subjects, to be irritating.

Another drawback is the fact that carboxytherapy treatments are very slow and tedious for some treated subjects.

In addition, it is important to further improve the treatment in terms of its effectiveness.

In this situation, the technical task underlying the present invention is to devise a device for the administration of carboxytherapy that is capable of substantially overcoming at least some of the above-mentioned drawbacks.

Within the sphere of said technical task, one important purpose of the invention is to obtain a device for the administration of carboxytherapy that is not considered to be irritating.

Another important purpose of the invention is to provide a device for the administration of carboxytherapy that enables quick treatments.

Not the least important purpose of the invention, is to provide an effective device for the administration of carboxytherapy.

The technical task and specified purposes are achieved by a device for the administration of carboxytherapy as claimed in the appended Claim 1.

The characteristics and benefits of the invention will be clarified in the following detailed description of some preferred embodiments of the invention, with reference to the accompanying drawings, wherein:

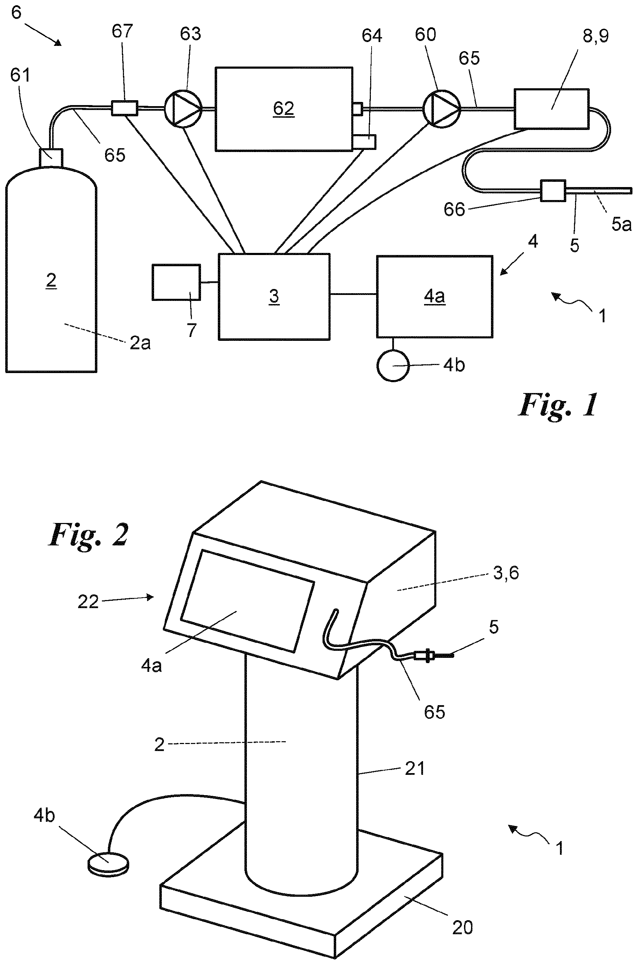

**Fig. 1** shows a diagram of the components of the device for the administration of carboxytherapy according to the invention; and
**Fig. 2** shows an example of a device for the administration of carboxytherapy. In the present document, the measures, values, shapes, and geometric references (such as perpendicularity and parallelism), when associated with words like "about" or other similar terms such as "approximately" or "substantially", are to be understood as except for measurement errors or inaccuracies owing to production and/or manufacturing errors and, above all, except for a slight divergence from the value, measure, shape, or geometric reference with which it is associated. For example, these terms, if associated with a value, preferably indicate a divergence of not more than 10% of the value.

Furthermore, when used, terms such as "first", "second", "higher", "lower", "main" and "secondary" do not necessarily identify an order, a priority relationship, or a relative position, but can simply be used to more clearly distinguish between the different components.

The measurements and data contained in the present text are to be considered, unless otherwise indicated, as carried out in ICAO International Standard Atmosphere (ISO 2533:1975).

With reference to the figures, the number **1** indicates, as a whole, the device for the administration of carboxytherapy according to the invention.

It comprises control means **3** and command means **4** for the device 1.

The control means 3 preferably consist of a circuit board, a computer, or the like, and are designed to electrically activate, deactivate, and command the elements and devices present.

The command means 4 are functionally connected to the control means 3 and are designed to enable the user to command the control means 3 and, therefore, the whole device 1. They conveniently comprise a touch screen **4a** and, preferably, a pedal **4b** as well.

The control means 3 and the command means 4 are preferably, at least in part, connected to a power supply system **7,** which preferably comprises a transformer and a connection to the power mains.

The device for the administration of carboxytherapy 1 comprises a pressure container **2** for carbon dioxide (CO₂) defining an internal volume **2a,** and an injection needle **5,** suitable for subcutaneous insertion. The injection needle 5 defines an internal channel **5a** that is in fluidic through connection with the internal volume 2a of the pressure container 2.

Between the pressure container 2 and the injection needle 5 there is a carbon dioxide channelling circuit **6**, to which the pressure container 2 and the injection needle 5 form the ends.

The pressure container 2 preferably consists of a replaceable cylinder that can be connected to a coupling **61**, which is part of the channelling circuit 6 and is known in itself.

The injection needle 5 is preferably of the disposable type and can, in turn, be connected to an end coupling **66**, which is known in itself.

The channelling circuit 6 also comprises an output valve **60**, suitable for opening or closing the channelling circuit 6.

The output valve 60 is suitable for opening or closing said channelling circuit 6 with an activation frequency greater than 0.5 Hz and enabling a flow greater than 100 cm³/min.

The activation frequency is adjustable and, more preferably, higher, having maximum values higher than 1 Hz, even more preferably higher than 5 Hz, even more preferably higher than 10 Hz, even more preferably close to 20 Hz, and preferably lower than 50 Hz.

Said activation takes place with duty cycles ranging between 0.2 and 0.8 and, preferably, between 0.4 and 0.6.

The maximum flow, during opening, of the valve 60, which passes through the internal channel 5a, is adjustable and preferably higher than 200 cm³/min, more preferably higher than 300 cm³/min, even more preferably higher than 500 cm³/min, and even more preferably higher than 800 cm³/min.

The output valve 60 is, preferably, a speed-up solenoid valve.

The channelling circuit 6 also comprises an intermediate tank **62** suitable for storing carbon dioxide. It is preferably arranged under pressure near the injection needle 5 and upstream of the output valve 60.

The intermediate tank 62 is preferably preceded, in the direction of the pressure container 2, by an inlet valve **63**, preferably a solenoid valve for opening and closing the connection to the container 2. The inlet valve 63 is preferably, in turn, preceded by a normally open pressure switch **67.**

The intermediate tank 62 also comprises a normally closed pressure switch **64**, which controls excess pressure of the same intermediate tank 62.

The intermediate tank 62 is preferably followed by the output valve 60 in the direction of the injection needle 5 along the channelling circuit 6.

In addition , heating means 8 are provided for the CO₂ flow downstream of the output valve. These preferably consist of an electrical resistance connected to the command means 4 and are suitable for heating the CO₂ fluid to desired temperatures. The heating means 8 are preferably arranged near the injection needle 5.

The portions of the channelling circuit 6 are then, preferably, mutually joined by means of pipes **65**, which are preferably flexible and made of polymer material. They are also, conveniently, easy to replace.

Finally, the device 1 comprises pressure regulation means **9** for the outlet pressure of the carbon dioxide leaving the internal channel 5a of the injection needle 5. The regulation means 9 are suitable for compensating the back pressure that the carbon dioxide flow encounters at the outlet of the internal channel 5a and, conveniently, for keeping the flow continuous, independently of the resistance. They consist of pressure measurement means and means for regulating the opening of the valves 60 and/or 63 to regulate the flow. They are also preferably arranged close to or at the heating means 8.

Structurally, the device for the administration of carboxytherapy 1 preferably comprises, as shown in the example in Fig. 2, a base **20**, for supporting the device and preferably equipped with wheels, a support column **21**, conveniently suitable for containing the pressure container 2 with carbon dioxide, and an upper portion **22.** The latter is at a manually controlled height, includes the control means 3, the command means, and the circuit 6, and is connected to the injection needle 5 and to the pedal 4b by means of flexible connections.

The operation of the device for the administration of carboxytherapy 1, previously described in structural terms, is as follows.

Initially, the pressure container 2 with carbon dioxide is connected to the coupling 61 and the injection needle 5 is mechanically connected to the end coupling 66.

The internal volume 2a of the pressure container 2, the internal channel of the pipes 65, the internal volume of the intermediate tank 62, and the internal channel 5a of the injection needle 5 are therefore in fluidic through connection and preferably pneumatically sealed from and to the outside.

A user can then use the device for the administration of carboxytherapy 1 by inserting the injection needle 5 subcutaneously in a subject to be treated. The user can then command the device for the administration of carboxytherapy 1 via the command means 4 connected to the control means 3.

In particular, the activation frequencies, the flow rate, possibly the duty cycles, the activation periods, and more, are set by the user via the touch screen 4a.

The carbon dioxide flows, due to the pressure, from the internal volume 2a of the pressure container 2 to the channelling circuit 6. The fluid passes through the inlet valve 63 and enters the intermediate tank 62 where it continues to enter, keeping it at the desired pressure.

When the user commands the opening of the output valve 60, preferably through the pedal 4b, the carbon dioxide flows through the pipes 65, is heated by the heating means 8, preferably reaching temperatures close to body temperature, and, more preferably, differing by no more than 5°C from the same body temperature.

The carbon dioxide finally flows through the internal channel 5a of the injection needle 5 and, preferably, enters subcutaneously into the treated subject, performing the carboxytherapy treatment.

The body of the treated subject or, in any case, the target of the treatment, creates back pressure when the carbon dioxide comes out of the internal channel 5a and enters the human body. This back pressure varies from individual to individual. It is measured by the regulation means 9 that regulate the outlet pressure of the carbon dioxide flow in such a way that the difference between the latter and said back pressure is constant. In this way, the outlet pressure is personalised according to the user and does not cause irritation to one person nor does it prove to be ineffective with others.

When the carbon dioxide enters the human body it does not enter with a constant flow but with an open/closed flow according to the activation frequencies described, and which can be set by the user, and also according to the duty cycles described and the flow values described, all of which are regulated.

The disclosure comprises a new method (not being part of the claimed subject-matter) of activation for a device for the administration of carboxytherapy 1, preferably of the type described above, comprising the opening and closing of the output valve 60 with a frequency greater than 0.5 Hz and with a flow greater than 100 cm³/min.

The method also includes, preferably, the features of the flow, frequencies, flow rates and duty cycle, previously described and, preferably, the operating modes previously described.

The method is preferably used for one or more of the following purposes: for the treatment of cellulite and localized adiposities, treatment of skin problems before and after surgery, revitalization of skin areas, vulvar rejuvenation, improvement of tissue oxygenation, improvement of circulation, treatment of arterial vessel dilation, treatment of arterial disease, treatment of microcirculatory stasis due to medium venous insufficiency in angiology, treatment of rheumatic diseases and periarthritis in orthopaedics.

The device for the administration of carboxytherapy 1 according to the invention entails important advantages.

In fact, the same device 1 is more effective in performing carboxytherapy, in particular because of the described open/closed flow, preferably regulated according to the activation frequencies and various features described.

In addition, the treatments, thanks in particular to the higher flow rate, are quicker. Another advantage is the fact that, thanks especially to the regulation means 9, which ensure that the difference between the outlet pressure of the carbon dioxide flow and the back pressure of the treated subject constant, the outlet pressure is personalised according to the user and, consequently, does not cause irritation to some patients nor does it prove to be ineffective with others.

The invention is susceptible to variations falling within the scope of the inventive concept as defined by the claim.

## Claims

1. A device for the administration of carboxytherapy (1) comprising:
- a pressure container (2) of carbon dioxide defining an internal volume (2a),
- control means (3) and command means (4) for said device (1),
- an injection needle (5), suitable for subcutaneous insertion and defining an internal channel (5a),
- a channelling circuit (6) in a fluidic through connection with said internal volume (2a) of said pressure container (2) and with said internal channel (5a) of said injection needle (5),
- said channelling circuit (6) comprising an output valve (60), suitable to open or close said channelling circuit (6), and an intermediate tank (62) suitable to store said carbon dioxide under pressure in proximity to said injection needle (5),
- pressure regulation means (9) suitable to measure a back pressure and to regulate the outlet pressure of the carbon dioxide flow in such a way that the difference between the latter and said back pressure is constant, so to compensate the back pressure which the carbon dioxide flow encounters at the outlet of said internal channel (5a) of said injection needle (5),
- heating means (8) for heating the flow of CO₂,
**characterized by** said output valve (60) being configured to open or close said channelling circuit (6) with an activation frequency adjustable and greater than 0.5 Hz with a flow adjustable and greater than 100 cm³/min, wherein said activation takes place with a duty cycle which is adjustable between 0.2 and 0.8.

## Patentansprüche

1. Vorrichtung für die Verabreichung von Carboxytherapie (1), umfassend:
- einen unter Druck stehenden Behälter (2) mit Kohlendioxid, der ein Innenvolumen (2a) definiert,
- Steuerelemente (3) und Bedienelemente (4) der genannten Vorrichtung (1),
- eine Injektionsnadel (5), die geeignet ist, subkutan eingeführt zu werden und einen inneren Kanal (5a) definiert,
- einen Kanalisierungskreis (6), der strömungstechnisch mit dem genannten Innenvolumen (2a) des genannten unter Druck stehenden Behälters (2) und mit dem genannten inneren Kanal (5a) der genannten Injektionsnadel (5) verbunden ist,
- wobei der genannte Kanalisierungskreis (6) ein Ausgangsventil (60) umfasst, das geeignet ist, den genannten Kanalisierungskreis (6) zu öffnen oder zu schließen, und einen Zwischenbehälter (62), der geeignet ist, das genannte unter Druck stehende Kohlendioxid in der Nähe der genannten Injektionsnadel (5) zu speichern,
- Druckregelelemente (9), die geeignet sind, einen Rücklaufdruck zu messen und den Ausgangsdruck des Kohlendioxidflusses so zu regeln, dass die Differenz zwischen Letzterem und dem genannten Rücklaufdruck konstant ist, um den Widerstandsdruck auszugleichen, auf den der Kohlendioxidfluss am Ausgang des genannten inneren Kanals (5a) der genannten Injektionsnadel (5) trifft.
- Wärmeelemente (8) des CO₂-Flusses,
- **dadurch gekennzeichnet, dass** das genannte Ausgangsventil (60) darauf ausgelegt ist, den genannten Kanalisierungskreis (6) mit einer regulierbaren Aktivierungsfrequenz von über 0,5 Hz und mit einem regulierbaren Fluss von über 100 cm³/min zu öffnen oder zu schließen, wobei die genannte Aktivierung bei einer Einschaltdauer zwischen 0,2 und 0,8 erfolgt.

## Revendications

1. Dispositif d'administration de carboxythérapie (1) comprenant :
- un récipient sous pression (2) de dioxyde de carbone définissant un volume interne (2a),
- des moyens de contrôle (3) et des moyens de commande (4) dudit dispositif (1),
- une aiguille d'injection (5), apte à être insérée sous la peau et définissant un canal interne (5a),
- un circuit de canalisation (6) en communication fluide avec ledit volume interne (2a) dudit récipient sous pression (2) et avec ledit canal interne (5a) de ladite aiguille d'injection (5),
- ledit circuit de canalisation (6) comprend une vanne de sortie (60), apte à ouvrir ou fermer ledit circuit de canalisation (6), et un réservoir intermédiaire (62) apte à stocker ledit dioxyde de carbone sous pression à proximité de ladite aiguille d'injection (5),
- des moyens de réglage de la pression (9) aptes à mesurer une pression de retour et à régler la pression de sortie du flux de dioxyde de carbone de sorte que la différence entre ce dernier et ladite pression de retour soit constante, de façon à compenser la pression de résistance qui s'oppose au flux de dioxyde de carbone au niveau de la sortie dudit canal interne (5a) de ladite aiguille d'injection (5).
- des moyens de chauffage (8) du flux de CO₂,
- **caractérisé en ce que** ladite vanne de sortie (60) est configurée pour ouvrir ou fermer ledit circuit de canalisation (6) avec une fréquence de déclenchement réglable et au-dessus de 0,5 Hz et avec un flux réglable et supérieur à 100 cm³/min, où ledit déclenchement survient avec un cycle de service compris entre 0,2 et 0,8.
